(19) **Europäisches Patentamt / European Patent Office / Office européen des brevets**

(11) **EP 4 359 811 B1**

(12) # EUROPEAN PATENT SPECIFICATION

(45) Date of publication and mention
of the grant of the patent:
**20.05.2026 Bulletin 2026/21**

(21) Application number: **22732590.9**

(22) Date of filing: **20.06.2022**

(51) International Patent Classification (IPC):
**G01R 33/565** (2006.01)    **A61B 5/055** (2006.01)
**G01R 33/28** (2006.01)    **G01R 33/563** (2006.01)

(52) Cooperative Patent Classification (CPC):
**G01R 33/56509**; G01R 33/283

(86) International application number:
**PCT/EP2022/066701**

(87) International publication number:
**WO 2022/268698 (29.12.2022 Gazette 2022/52)**

(54) **DETERMINATION OF 3D POSITIONING DATA IN AN MRI SYSTEM**

BESTIMMUNG VON 3D-POSITIONSDATEN IN EINEM MRT-SYSTEM

DÉTERMINATION DE DONNÉES DE POSITIONNEMENT 3D DANS UN SYSTÈME D'IRM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.06.2021 EP 21180516**

(43) Date of publication of application:
**01.05.2024 Bulletin 2024/18**

(73) Proprietor: **Koninklijke Philips N.V.
5656 AE Eindhoven (NL)**

(72) Inventors:
 • **SOMMER, Karsten
  5656 AG Eindhoven (NL)**
 • **KRUEGER, Sascha
  5656 AG Eindhoven (NL)**
 • **WUELBERN, Jan Hendrik
  5656 AG Eindhoven (NL)**
 • **GRAESSLIN, Ingmar
  5656 AG Eindhoven (NL)**
 • **FRERKING, Lena Christina
  5656 AG Eindhoven (NL)**

(74) Representative: **Philips Intellectual Property &
Standards
High Tech Campus 52
5656 AG Eindhoven (NL)**

(56) References cited:
**US-A1- 2018 325 415    US-A1- 2020 205 748**

 • **KYME ANDRE Z. ET AL: "Marker-free optical
stereo motion tracking for in-bore MRI and PET-
MRI application", MEDICAL PHYSICS., vol. 47,
no. 8, 1 June 2020 (2020-06-01), US, pages 3321 -
3331, XP055846660, ISSN: 0094-2405, DOI:
10.1002/mp.14199**
 • **KYME ANDRE ET AL: "Markerless Motion
Tracking of Awake Animals in Positron Emission
Tomography", IEEE TRANSACTIONS ON
MEDICAL IMAGING, IEEE, USA, vol. 33, no. 11, 1
November 2014 (2014-11-01), pages 2180 - 2190,
XP011562852, ISSN: 0278-0062, [retrieved on
20141028], DOI: 10.1109/TMI.2014.2332821**
 • **HENRY DAVID ET AL: "Non-rigid Motion
Detection for Motion Tracking of the Head", 2019
IEEE NUCLEAR SCIENCE SYMPOSIUM AND
MEDICAL IMAGING CONFERENCE (NSS/MIC),
IEEE, 26 October 2019 (2019-10-26), pages 1 - 3,
XP033747762, DOI: 10.1109/NSS/
MIC42101.2019.9059653**
 • **MAXIM ZAITSEV: "Hardware Methods for
Handling Motion: Basic Ideas & Current
Methods", INTERNATIONAL SOCIETY FOR
MAGNETIC RESONANCE IN MEDICINE, ISMRM,
2030 ADDISON STREET, 7TH FLOOR,
BERKELEY, CA 94704 USA, no. 2547, 15 May
2015 (2015-05-15), XP040668224**

- THOMAS SIEGERT, ENRICO REIMER, ROLAND MÜLLER, ROBERT TURNER, HARALD MÖLLER, AND JESSICA SCHULZ: "First experiences with a Time of Flight (ToF) Camera for marker-less motion tracking within a 7 Tesla MR scanner", INTERNATIONAL SOCIETY FOR MAGNETIC RESONANCE IN MEDICINE, ISMRM, 2030 ADDISON STREET, 7TH FLOOR, BERKELEY, CA 94704 USA, no. E1124, 1 June 2018 (2018-06-01), XP040704926

**Description**

FIELD OF THE INVENTION

The present invention relates to a method for motion correction of MR data, an apparatus for motion correction of MR data, a system for medical imaging, and computer program element.

BACKGROUND OF THE INVENTION

Magnetic resonance imaging, MRI, is known from the state of the art. MRI is used to obtain medical images of an anatomy, for example an organ of a human. MRI uses strong magnetic fields, magnetic field gradients, and radio waves for obtaining medical images. The quality of the medical image is crucial for a comparison with a reference image or for analysis of the medical image (e.g. determining of certain areas in an organ, etc.). The quality of the medical image depends among others on an alignment of desired object to be imaged and an imaging system. The information of the alignment sometimes may be incorrect or may change over the time and may therefore have negative effects on the quality of the resulting medical image.

The article "Marker-free optical stereo motion tracking for in-bore MRI and PET-MRI application" by A. Kyme et al., Medical Physics, vol. 47, no. 8 (1 June 2020) discloses a method for prospective motion correction.

The article "Markerless motion tracking of awake animals in positron emission tomography" by A. Kyme et al., IEEE transactions on medical imaging, vol. 33, no. 11 (1 November 2014) discloses a method for motion-compensation in positron emission tomography.

United States patent application US 2018/0325415 A1 discloses a method for measuring motion information from a human or animal subject during a medical imaging examination.

SUMMARY OF THE INVENTION

There may, therefore, be a need for an improved determining of a position of a subject in an MRI system. The object of the present invention is solved by the subject-matter of the independent claims, wherein further embodiments are incorporated in the dependent claims.

According to a first aspect, a method for motion correction of MR data is provided, comprising: generating, by a calculation unit, a three-dimensional model, 3D model, of a region of interest of a subject comprising at least one landmark inherent to the subject. The method further comprises obtaining, by a first measuring device, a two-dimensional image, 2D image, of at least a part of the subject inside a MRI system, wherein the measuring device is arranged at least partially inside a bore of the **MRI** system. In addition, the method comprises determining, by the calculation unit, at least one landmark in the 2D image, wherein the at least one landmark in the 2D image corresponds to the at least one landmark of the 3D model. The method further comprises determining, by the calculation unit, a position of the region of interest of the subject in the MRI system based on the determined at least one landmark in the 2D image and providing, by the calculation unit, the 3D position of the region of interest of the subject for motion correction of MR data.

The term MR data, as used herein, is to be understood broadly and relates to data associated with the MRI procedure. The MR data may relate to data used to control the MRI system in a preparation phase of the MRI procedure and/or in an operating phase of the MRI procedure. The MR data may comprise data and/or information used to generate an adaption signal and/or control signal for magnetic resonance gradients, radiofrequency pulses, and receiver frequency in a scanner of the MRI system. The motion correction of MR data in a preparation phase and/or operating phase of the MRI procedure is also known as prospective motion correction. Thereby, the preparation phase may precede the operating phase. The MR data may relate to MR images obtained, e.g. acquired, with the MRI procedure. The motion correction of MR images in a post processing phase of the MRI procedure is also known as retrospective motion correction.

The term calculation unit, as used herein, is to be understood broadly and means a unit configured to process data, in particular to determine one or more landmarks in a 2D image by data processing. The calculation unit may be a hardware unit (e.g. a controller, a workstation, a server) or a software unit (e.g. a virtual machine executed on a hardware unit), or a combination of hardware and software. The control unit may be in a single entity or distributed on several entities, wherein an entity may be a hardware unit and/or a software unit.

The term 3D model, as used herein, is to be understood broadly and relates to a model configured to describe a region of interest of a subject. The 3D model may be a point model, a line model, a surface model, or a volume model. The 3D model may, for example, comprise information of facial features, bones, tissues, organs, and/veins, wherein other body parts, organs, etc. may also be taken as information. The 3D model may be based on statistical information from e.g. a database or the like, considering e.g. age, sex, weight, or the like. For example, the 3D model may be based on an anatomy atlas. The 3D model may also be based on historical data from a subject to be imaged (e.g. previous images from a previous medical imaging exam). The 3D model may be described with e.g. a vector, wherein the vector may comprise 3D information of the at least one landmark, preferably of several landmarks.

The term subject, as used herein, means a human or animal. The region of interest, as used herein, relates to any part of a human or animal body (e.g. bone, tissue, organ, or combination thereof, wherein other body parts, organs, etc. may also be taken as information).

The term landmark, as used herein, is to be understood broadly and relates to at least one marker configured to be determined in a 2D image and an image with 3D data. The landmark may be a structural, e.g. inherent, component of the region of interest (e.g. a bone, eye, nose, hand etc.), a color combination or color transition (e.g. mole on left cheek), or a virtual marker adjacent to the region of interest (e.g. derived from two or more physical markers of the region of interest, e.g. two bones). The landmark is in particular not a separate entity that is attached to the subject, but is rather inherent to the subject. The landmark may be part of the subject or of the adjacent area visible in the 2D image. The landmark may be determined by an image analysis algorithm, for instance an edge detection algorithm. The landmark may be determined automatically by the image analysis algorithm in the 2D image, wherein the 2D image is continuously obtained by a video stream from an in-bore camera. In particular, the at least one landmark in the 2D image may be obtained by searching for the at least one landmark of the 3D model. As an example, the image analysis algorithm may specifically scan the 2D image for certain features of the at least one landmark of the 3D model. As another example, the image analysis algorithm may find landmarks in the 2D image and then match them to the at least one landmark of the 3D model. The landmark may preferably be predefined. In case more than one landmark is predefined, for example five, and inside the bore of the MRI system only e.g. four landmarks are determined, the method may continue the execution with the e.g. four landmarks.

The term first measuring device, as used herein, is to be understood broadly and relates to any measuring devices configured to obtain a 2D image of a part of a subject inside a bore of a MRI system. The first measuring device may be a sensor unit. The measuring device may be an optical camera sensor, an infrared sensor, a laser interferometer, or the like. The first measuring device may be one single entity or distributed on two or more entities, wherein an entity relates to a senor unit. The first measuring device may, for example, be an RGB sensor or a CCD sensor. The first measuring device may be an infrared camera or the like, continuously streaming from inside the bore. The first measuring device may obtain 2D images continuously or after discrete times. The first measuring device may be arranged in direct visual contact to the subject, in particular the part of the subject, more particularly the region of interest of the subject. The first measuring device may be arranged in indirect visual contact by means of a mirror to the subject, in particular the part of the subject, more particularly the region of interest of the subject. The first measuring device may be in wired connection (e.g. Ethernet) with the calculation unit, data storage, a server, a workstation. The first measuring may be in wireless in connection with the abovementioned entities (e.g. WIFI).

The MRI system, as used herein, relates to a state of the art MRI system configured to execute an MRI procedure. The MRI system, as used herein, comprises at least an MRI control configured to control the MRI system, a bore in which a movable support structure bearing a subject is positioned, one or more MR source coils, one or more MR detection coils. The MRI system may advantageously enhance by means of a first measuring device.

The term 3D position, as used herein, means at least the three translatory coordinates (e.g. x, y, z coordinates) of a single point of the region of the interest of the subject. The 3D position of one or more single points may reveal an orientation of the region of interest of the subject. The 3D position may comprise further three rotational coordinates.

The invention is based on the finding, that the knowledge of the 3D position of a subject, in particular, the knowledge of the position of the region of interest of the subject in relation to the MRI system (e.g. expressed in MRI system coordinates) in an MRI procedure is crucial for a quality of a resulting MR image. The resulting measuring data from the MRI procedure have to be further processed in order to obtain the MR image. The further processing for obtaining the MR image requires the 3D position of the region of interest of the subject. In case the used 3D position of the region of interest of the subject in further processing for obtaining the MR image is inaccurate, the resulting MR image is also inaccurate. The 3D position of the region of interest of the subject may change over the time, as the subject may inhale, exhale or simply moves. However, the changed 3D position of the region of interest will influence the meta data of MRI procedure, and therefore the corresponding obtained MR image in case no motion correction is carried out. The invention determines the 3D position of the region of interest of the subject inside the bore of the MRI system and uses the 3D position of the region of interest of the subject to correct the MR images after they were imaged (i.e. retrospective motion correction). Furthermore, the determined 3D position of the region of interest of the subject may also be used to adapt the MRI procedure, in particular the magnetic resonance gradients, radiofrequency pulses, and receiver frequency of MRI system before further MRI procedure (i.e. prospective motion correction). The determining of the 3D position of the region of interest of the subject is carried by a 2D image obtained by a state of the art measurement device, in particular a 2D camera sensor implemented inside the bore of MRI system. This may be advantageous as only a single 2D camera, in particular no 3D depth camera, is required. The 2D camera may be advantageously operated inside the magnetic field inside the bore of the MRI system in comparison to a 3D camera, e.g. depth camera. The invention enables the use of the simple but robust 2D camera by mapping information from the obtained 2D image to the 3D model and thereby determining the 3D position of the region of interest of the subject. The invention enables Magnetic resonance motion artefact correction using a single in-bore camera and an additional depth camera arranged outside the bore (e.g. in the scanner room).

The method further comprises obtaining at least one modelling image of the subject, by a second measuring device arranged outside the bore of the medical imaging system, wherein the at least one modelling image is used for generating the 3D model of the region of interest of the subject. The term modelling image, as used herein, means that modelling is merely used for generating the 3D model of the region of interest of the subject in a preparation phase of the MRI procedure. For instance, a subject may lie on a support structure outside the bore of an MRI system and the second measuring device is arranged over the subject (e.g. at the ceiling). The measuring device may take one or more modelling images of the region of interest from one or more perspectives. The 3D model may comprise a default 3D model with default dimensions which is adapted by the at least one modelling image of the region of interest of the subject. Actual dimensions of the region of interest of the subject may directly be obtained from the modelling image by analysis of the modelling image. The analysis may comprise utilizing e.g. a neural network in case the modelling image comprises merely 2D data. The analysis may comprise simply a reading from the modelling image in case the modelling image comprises 3D data. The 3D model may be newly generated, e.g. during performing the method described herein, etc., which means that no default 3D model is present. In sum, this may be advantageous as the accuracy of the 3D model may increase and therefore the resulting quality of the MR data. The modelling image may be continuously received from the second measuring device. The modelling image may be continuously analyzed to detect the at least one landmark, wherein the analyzing may comprise one or more mathematical algorithms. The at least one landmark may be predefined for the region of interest (e.g. a cheek bone for the head). The mathematical algorithm may comprise an edge detection algorithm, a neural network trained for this purpose, or other suitable calculation methods. The mathematical algorithm may determine whether the subject is on a bed of the MRI system, and/or whether the subject is outside or inside the bore of MRI system. The mathematical algorithm may determine different parts of the subject (e.g. head, leg, arm, etc.). The mathematical algorithm may use any data stream from the second measuring device (e.g. 2D information (RGB output from depth camera), 3D information from a depth camera or combination thereof). The mathematical algorithm may first detect a predefined region of interest (e.g. head) and then a predefined at least one landmark (e.g. a cheek bone). In case more than one modelling image is obtained, generating the 3D model of the region of interest of the subject may comprise the calculation of an average 3D model. In case more than one modelling image is obtained, generating the 3D model of the region of interest of the subject may comprise a merger of different 3D models of the region of interest of the subject.

In an embodiment, the at least one modelling image may comprise 3D data of the region of interest of the subject. The 3D data may comprise for example translatory coordinates (x, y, z direction) of each pixel in the modelling image. The 3D data may be obtained from a depth camera, a laser interferometer scanner, and/or two or more 2D cameras (e.g. two RGB sensor cameras in different perspectives that enable computer stereo vision). Computer stereo vision is the extraction of 3D information from digital images. By comparing information from a region of interest from two perspectives, 3D data can be extracted by examining relative positions of objects (e.g. landmarks) in the digital images. The 3D data of the region of interest of the subject may advantageously increase the accuracy of the 3D model and therefore of the resulting MR data. The 3D data from the modelling images may be used to obtain corresponding 3D spatial positions in a suitable coordinate system (e.g. MRI coordinate system, region of interest (e.g. head) coordinate system). For instance, a patient-centered coordinate system may be used, where the at least one landmark is the origin of the coordinate system. The coordinate system may comprise homogenous coordinates in order to simplify the calculation.

In an embodiment, the second measurement device may be a depth camera. The depth camera may advantageously provide very accurate 3D information data of the region of interest of the subject. The depth camera may use a time of flight principle to determine 3D information from a 2D image. The depth camera may use structured light to determine 3D information from a 2D image. The depth camera may use coherent light and measure a phase shift between of reflected light relative to a source light (i.e. laser interferometry).

In an embodiment, the second measuring device may be at least one optical camera. The optical camera may be a RGB camera. The RGB camera provides merely a 2D image. Hence, either two images from two different perspectives are necessary to derive 3D data from an RGB camera (i.e., computer stereo vision, which preferably requires at least two optical cameras) or the 2D data from the 2D image obtained by the RGB camera has to be aligned to a 3D model. The second option may be carried out by means of a mathematical algorithm. The mathematical algorithm may be trained to process one or more inputs into one or more outputs by means of an internal processing chain that typically has a set of free parameters. The internal processing chain may be organized in interconnected layers that are traversed consecutively when proceeding from the input to the output. The mathematical algorithm may be trained by using records of training data. A record of training data comprises training input data and corresponding training output data. Training input data, as used herein, may be 2D images from the region of interest of the subject and training output data may be 3D data of the region of interest of the subject (e.g. measured with a 3D depth camera). The training input data and training output data may also be simulated data in order to reduce the effort of providing training data. In sum, this may advantageous in terms cost reduction and accuracy of the 3D model. The optical camera may be an infrared camera. The optical camera may use a charged-coupled device (CCD) sensor or an active-pixel sensor (i.e. complementary metal-oxide-semiconductor (CMOS) sensor).

In an embodiment, the generating of the 3D model may be based on a machine learning system representing a mathematical algorithm processing at least one landmark of a region of interest of a subject, wherein the machine learning system is trained to describe a relation between geometrical data of a region of interest of a subject and at least one landmark of the region of interest of the subject. The training data may be derived from records of 2D images and corresponding 3D images. The 2D images and corresponding 3D images may be simulated in order to reduce the effort providing training data. The machine learning system may be implemented by a neural network, machine learning algorithm, convolutional neural network, or a generative adversarial network. The use of the machine learning system may be advantageous in terms of efficiency and accuracy of generating the 3D model.

In an embodiment, the determining of the position of the region of interest of the subject in the MRI system may comprise determining of one or more rotations and one or more translations of the 3D model in relation to a position of the first measuring device in order to obtain a projection of the 3D model that fits to the 2D image obtained by the first measuring device inside the bore. In other words, the method checks what 3D position of the region of interest of the subject may lead to the obtained projection of the region of interest of the subject (i.e. 2D image). The determining therefore may further consider the position of the first measuring device inside the bore. The determining may calculate in reference from the position of the first measuring device inside the bore a perspective. The determining may use physical equations of intercept theorems. The determining may use numerical approximation method to determine the position of the subject in the MRI system. The determining may further use a neural network, which may be trained for this purpose, to determine the position of the subject in the MRI system. In sum, this may be advantageous to accurately determine the position the subject in the MRI system.

In an embodiment, the first measuring device may be arranged in a coil, or in housing or the like thereof. The arrangement of the first measuring device in a coil may be advantageous as nothing may hide or obscure the view of between the measuring device and the region of interest of the subject. The coil may be a head coil, or another body coil. The coil may be a stationary coil inside of MRI system. The first measuring may also be mounted on the ceiling of the bore. The first measuring device may also change its perspective in dependency of the subject and/or the region of interest (e.g. different sizes of the subject require different perspectives). The measuring device may also be arranged in combination with one or more mirrors in order to image concealed areas (e.g. underside chin). In case a mirror is used also the position of the mirror is used beside the position of the measuring device for determining the position of the subject in the MRI system.

In an embodiment, the modelling image may be used to obtain the corresponding 3D positions in a suitable coordinate system. For example, a patient-centered coordinate system may be used, where one landmark of a plurality of predefined landmarks is taken to be the origin. Since multiple modelling images of the region of interest may usually be available during an exam preparation, the resulting 3D model can be averaged and stitched to improve accuracy. Using homogeneous coordinates, the resulting individual reference landmark positions may be described by a vector $\vec{r}_i = \begin{pmatrix} x_i \\ y_i \\ z_i \\ 1 \end{pmatrix}$. To describe the 3D model, these positions may be aggregated into a single reference vector $\widetilde{\vec{r}}^{\,\mathrm{ref}} = \begin{pmatrix} \vec{r}_0 \\ \vec{r}_1 \\ \vdots \end{pmatrix}$.

Once the subject has been moved into the bore, the one or more landmarks are determined on the 2D image inside the bore of the MRI system. In case only a subset of the plurality of landmarks are determined with high accuracy, all calculations may be restricted to this subset of landmarks by simply dropping the non-detected landmark variables. The detected in-bore landmark positions may be described by (again in homogeneous coordinates) a vector $\vec{p}_i = \begin{pmatrix} w_i \\ v_i \\ 1 \end{pmatrix}$. A further in bore 2D model may be obtained by aggregating these vectors to $\widetilde{\vec{p}} = \begin{pmatrix} \vec{p}_0 \\ \vec{p}_1 \\ \vdots \end{pmatrix}$. The parameters describing the transformation of the reference 3D vector to the in-bore 2D vector are then found by solving:

$$\min_{\theta} \left\| \widetilde{\vec{p}} - CM_\theta \widetilde{\vec{r}}^{\,\mathrm{ref}} \right\|_2$$

$C$ is a block-diagonal matrix consisting of $3 \times 4$ matrices that describe the projection realized by the in-bore camera, and $M_\theta$ is a block-diagonal matrix describing the 3D transformation from the reference vector to the 3D vector inside the bore (parametrized by $\theta$). For a rigid motion model, $M_\theta = T_{\theta 1} R_{\theta 2}$, where $T_{\theta 1}$, and $R_{\theta 2}$ are the 3D translation and rotation matrix, respectively. For the first images acquired by the in-bore camera, the equation is solved using an initialization of $\theta$ corresponding to the

ideal pose of the region of interest (e.g. the head). The resulting transformation parameters $\theta^{(0)}$ are then used as initial values for all following in-bore camera images, which in turn yield $\theta^{(i)}$. The desired 3D positions are calculated as the deviation from the reference transformation:

$$\Delta\boldsymbol{\theta} = \boldsymbol{\theta}^{(i)} - \boldsymbol{\theta}^{(0)}$$

These 3D positions may then be further used for motion correction. In case of retrospective motion correction, the parameters may be stored time synchronized with the acquired MR data profiles for each acquired camera image of a particular MR scan, and sent to the reconstruction software once the scan is completed. In case of prospective motion correction, the calculated motion parameters may be directly sent to the scanner software to adjust the Magnetic resonance gradients, radiofrequency pulses, and receiver frequency and phase to account for the motion.

In an embodiment, the first measuring device may be an optical camera. The optical may be an RBG camera. The RGB camera is a measuring device with a robust operating behavior in magnetic environments such a MRT system. This may be advantageous in terms of robustness and reliability of the method. The optical camera may be an infrared camera. This may also be advantageous in terms of robustness and reliability of the method. It shall be noticed here that a single optical camera as first measuring device may be sufficient to execute the method. However, it may also be advantageous to use more than RGB camera in the bore in order to get a better coverage of the region of interest of the subject in the MRI system.

In an embodiment, the region of interest of the subject may be a head of the subject.

In an embodiment, generating the 3D model of the subject may be based on an anatomical model, preferably a 3D morphable model. This may be advantageous in terms of the accuracy of the 3D model. The 3D morphable model may be advantageous in case the region of interest is a head or a face. The 3D morphable model is a generative model, which may be established on set of example faces or heads in a registration procedure. The 3D morphable model may be a statistical model of a distribution of the example faces or heads.

In an embodiment, the position of the region of interest of the subject may continuously be determined and provided for the motion correction of the MR data. The continuous execution of the method may require real time capable hardware components for calculation and data exchange. The calculation may be carried out by out by a work station, a FPGA, high-performance computer, a data center. The data exchange between first measuring device, second measuring device, control of the MRI system, and the calculation unit may be carried out by a third generation Bus system, Ethernet, and a Fast-Ethernet-Hub.

According to a further aspect, an apparatus for motion correction of MR data is provided, comprising: a generating unit, configured to generate a 3D model of a region of interest of a subject comprising at least one landmark; an obtaining unit, configured to obtain a 2D image of at least a part of the subject inside a MRI system, wherein the obtaining unit is arranged inside a bore of the MRI system; a first determining unit, configured to determine at least one landmark in the 2D image, wherein the at least one landmark in the 2D image corresponds to the at least one landmark of the 3D model; a second determining unit, configured to determine a position of the region of interest of the subject in the MRI system based on the determined at least one landmark in the 2D image; a providing unit, configured to provide the position region of interest of the subject for a motion correction of MR data. The generating unit, the first determining unit, the second determining unit, the providing unit may be separate hardware units or separate software units that run on one or more hardware units, or combinations thereof. The hardware unit may be a controller, a computer, a server, a workstation. The data exchange between the one or more hardware units may wired (e.g. Ethernet, Profinet) or wireless (e.g. WIFI, WLAN).

According to a further aspect, a system for medical imaging is provided, comprising: an apparatus described above; an MRI system; a first camera, configured to obtain a 2D image inside a MRI system; and optionally a second camera, configured to obtain an image outside the MRI system. The second camera may be depth camera. The first camera may be a RGB camera.

According to a last aspect a computer program element is provided, which when executed by a processor is configured to carry out the steps of a method described above. The processor may be part of the medical imaging system, or may be provided separately in another computer device. The computer program element might be stored on a computer unit, which might also be part of an embodiment. This computing unit may be configured to perform or induce performing of the steps of the method described above. Moreover, it may be configured to operate the components of the above described device. The computing unit can be configured to operate automatically and/or to execute the orders of a user. A computer program may be loaded into a working memory of a data processor. The data processor may thus be equipped to carry out the method according to one of the preceding embodiments. This exemplary embodiment of the invention covers both, a computer program that right from the beginning uses the invention and computer program that by means of an update turns an existing program into a program that uses invention. Further on, the computer program element might be able to provide all necessary steps to fulfill the procedure of an exemplary embodiment of the method as described above. According to a further exemplary embodiment of the present invention, a computer readable medium, such as a CD-ROM, USB stick or the like, is presented wherein the computer readable medium has a computer program element stored on it which computer program element is described by the preceding section. A computer program may be stored and/or distributed on a suitable medium, such as an optical storage medium or a solid state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that the above embodiments may be combined with each other irrespective of the aspect involved. Accordingly, the method may be combined with structural features of the device and/or system of the other aspects and, likewise, the device and the system may be combined with features of each other, and may also be combined with features described above with regard to the method.

These and other aspects of the present invention will become apparent from and elucidated with reference to the embodiments described hereinafter.

BRIEF DESCRIPTION OF THE DRAWINGS

Exemplary embodiments of the invention will be described in the following drawings.

> Fig. 1 shows a schematic view of a part of a system for medical imaging according to an embodiment of the present disclosure;
> Fig. 2 shows a schematic view of an apparatus according to a further embodiment of the present disclosure; and
> Fig. 3 shows a flow chart diagram of a method for motion correction of MR data according to a further embodiment of the present disclosure.

DETAILED DESCRIPTION OF EMBODIMENTS

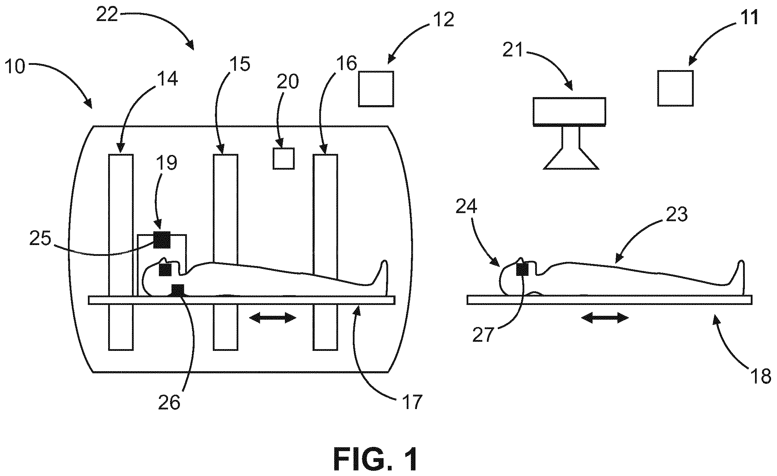

Fig. 1 shows a schematic view of a part of a system for medical imaging according to a first embodiment of the present disclosure.

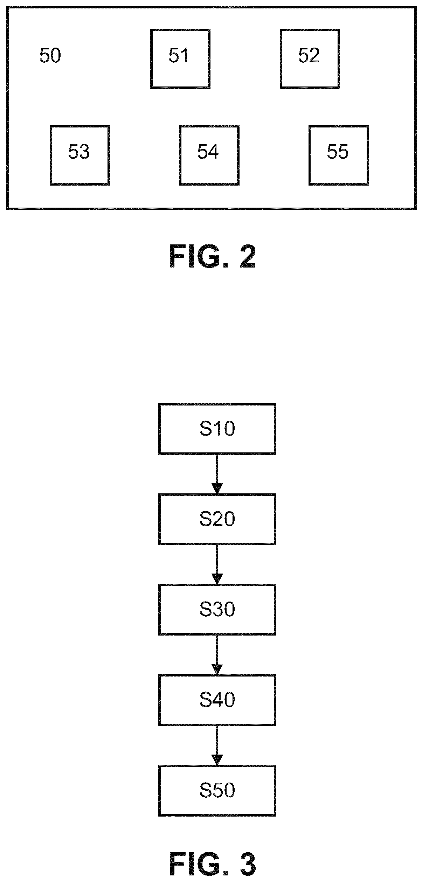

The system 10 for medical imaging comprises an apparatus 11 (see Fig. 2) for motion correction of MR data. The system 10 comprises further a MRI system 22, comprising a MRI controller 12, a bore 13, three magnetic coils 14, 15 and 16, a movable bed 17 and 18, a first camera 20 and 25, a second camera 21. The second camera 21 is configured to obtain an image outside the MRI system. The second camera is in the present case mounted at a ceiling over the movable bed 18. The second camera 21 is a depth camera that is able to provide images with 3D information data of region of interest 24 of the subject 23. Alternatively, instead of a depth camera also laser interferometer scanner could be used to obtain 3D information data of a region of interest 24 of the subject 23. Optionally, instead also two or more 2D cameras (e.g. RGB camera) could be used to obtain 3D data of the region of interest 24 of the subject 23. The subject 23 is in the present example a human. Optionally, the subject 23 could also be instead an animal, e.g. a shepherd dog, or the like. The region of interest 24 is in the present example the head of the subject 23. Optionally, instead any other area such as stomach, chest, arm, leg etc. could serve as region of interest. The region of interest is the area to be imaged by the MRI procedure. The subject 23 lays on a movable bed outside MRI system 22. The region of interest 24 comprises one landmark 27, in the present case a cheekbone. Optionally, the region of interest may also comprise more than one landmark. Optionally, the landmark may also be a mole on the face, an ear, an eye, a nose etc. Optionally, the landmark may a virtual marker adjacent to the region of interest, wherein the virtual landmark is derived from two or more physical landmarks of the region of interest (e.g. two cheek bones). The second camera 21 is in wired connection (e.g. Ethernet) with the apparatus 11. By means of the wire connection among others control signals and image data (i.e. 2D data and/or 3D data) is exchanged. In a preparation phase of the MRI procedure one or more modelling images of the subject 23 are obtained by the second camera 21. The apparatus 12 may control or trigger the imaging process of the second camera 21. When the preparation phase is finished the movable bed 17 on which the subject 23 lays, moves into the bore 13 of the MRI system 22. Optionally, a head coil 19 is arranged at the head 24 of the subject. Inside the bore 13 three source and detector magnetic coils 14, 15 and 16 are arranged on an inner surface of the bore 13. Inside the bore 13 a first camera 25 is arranged in the present example in a head coil 19. The first camera 25 is configured to obtain a 2D image inside the MRI system from the region of interest 24, in particular the head of the subject. The first camera 25 may be complemented with a mirror 26 that may be arranged adjacent to the head 24 of the subject in order to image concealed areas of the region of interest 24. Optionally, instead, the first camera 20 could be arranged on an inner surface of the bore 13. It should be noted that only one first camera is necessary. However, optionally, also more than one first could be arranged inside the bore in order to cover the whole area inside the bore. The first camera 20, 25 is a 2D RGB camera that generates 2D images. The first camera is in wired or wireless connection, preferably wired connection with the apparatus 11. The apparatus 12 may control or trigger the imaging process of the first camera 20, 25. The apparatus 12 may be in wired connection with the MRI control 12 in order to exchange data.

Fig. 2 shows a schematic view of an apparatus 50 according to a further embodiment of the present disclosure. The apparatus 50 is configured for motion correction of MR data of MRI system. The apparatus 50 comprises a generating unit 51. The generating unit 51 is configured to generate a 3D model of a region of interest of the subject comprising at least one landmark. The generating unit is in the present example a software unit implemented on a hardware unit. The hardware unit is in the present case a CPU of workstation. The generating unit 51 provides an anatomical model, in particular a 3D morphable model. The generating unit 51 receives from the second camera 21 one or more modelling images of the region of interest 24 of the subject 23 comprising the landmark 27. The data exchange between the generating unit 51 and the second camera 21 is established by an Ethernet connection. The generating unit 51 unit processes the one or more modelling images, wherein processing means determining a position of the landmark and determining sizes of the region of interest 24 of the subject 27, in order to generate the 3D model of the subject 27. The apparatus 50 comprises further an obtaining unit 52. The obtaining unit 52 is configured to obtain a 2D image of at least a part of the subject inside MRI system 22, wherein the obtaining unit 52 is arranged inside a bore of the MRI system. The obtaining unit 52 is in the present example the first camera 20 or 25 (see description Fig. 1). The apparatus comprises further a first determining unit 53. The first determining unit 53 is configured to determine at least one landmark in the 2D image, wherein the at least one landmark in the 2D image corresponds to the at least one landmark of the 3D model. The first determining unit 53 is in the present example a software unit implemented on the same hardware unit as the generating unit 51. The software unit may comprise an image processing software module in order to analyse the 2D image. The first determining unit 53 transmits the information of the determined at least one landmark to the second determining unit 54. The second determining unit 53 is part of the apparatus 50. The second determining unit 54 is in the present case a software unit implemented on the same hardware unit as the generating unit 51 and first determining unit 53. The second determining unit 54 is configured to determine a position of the subject in the MRI system based on the determined at least one landmark in the 2D image. The second determining unit 54 may comprise a mathematical algorithm trained to derive from 2D image the 3D position of the region of interest of the subject. The mathematical algorithm will be explained more in detail in Fig. 3. The apparatus 50 comprises further a providing unit 55. The providing unit 55 is in the present example a software unit implemented on the same hardware unit as the generating unit 51. The providing unit 55 is configured to provide the position of region of interest 24 of the subject 27 for a motion correction of MR data. The providing unit 53 may provide the position of the region of interest 24 of the subject 27 to the MRI controller 12 in order to adjust MRI procedure settings or to a server (not shown) in order to correct already obtained MRI images.

Fig. 3 shows a flow chart diagram of method for motion correction of MR data according to a further embodiment. The method comprises five steps. In a first step S10 a 3D model of a region of interest of a subject comprising at least one landmark is generated. The 3D model is generated by the generating unit 51 described above. Step S10 may further comprise obtaining at least one modelling image of the subject. The at least one modelling image may be obtained by the second camera 21, for example a depth camera. The at least one modelling image shows a region of interest of a subject, wherein the modelling image is obtained outside a bore of MRI system. The at least one modelling image may comprise 3D data. The 3D data comprise for example translatory coordinates (x, y, z direction) of each pixel in the modelling image. The 3D model may be based on a machine learning system representing a mathematical algorithm processing at least one landmark of a region of interest of a subject, wherein the machine learning system is trained to describe a relation between geometrical data of a region of interest of a subject and at least one landmark of the region of interest of the subject. The 3D model may further be an anatomical model, preferably a 3D morphable model. In a step S20 a 2D image of at least a part of the subject inside a MRI system is obtained by a first measuring device 20, 25, wherein the first measuring device is arranged inside a bore of the MRI system. The first measuring device may be RGB camera. The first measuring device may be arranged on an inner surface of bore or in a coil, in particular a head coil. The first measuring device may be complemented by a mirror. The 2D image may be obtained continuously, for instance every single second, in order to detect movements of the region of interest of the subject. In a step S30 at least one landmark 27 in the 2D image, wherein the at least one landmark in the 2D image corresponds to the at least one landmark of the 3D model, is determined. The determining of the at least one landmark is carried by an image analysis algorithm (e.g. edge detection). The image analysis algorithm may be trained with records from historical examinations. The records may comprise a plurality of landmarks (e.g. special bone, nose, eye, etc.). The image analysis algorithm may receive from the generating unit 51 the information of the at least one landmark which it must search for. In a step S40 a position of the region of interest of the subject in the MRI system based on the determined at least one landmark in the 2D image is determined. The determining is executed by second determining unit 54. The determining of the position of the subject in the MRI system may comprise determining of one or more rotations and one or more translations of the 3D model in relation to a position of the first measuring device in order to obtain a projection of the 3D model that fits to the 2D image obtained by the first measuring device inside the bore. The determining therefor may further consider the position of the first measuring device inside the bore. The determining may calculate from the position of the first measuring device inside the bore a perspective. The determining may use physical equations of intercept theorems. The determining may use a numerical approximation method to determine the position of the region of interest of the subject in the MRI system. The determining may further use a neural network to determine the position of the region of interest of the subject in the MRI system. In a step S50 the 3D position of the region of interest of the subject for motion correction of MR data is provided. The 3D position is either transmitted to the MRI control of the MRI system in order to correct the magnetic resonance gradients, radiofrequency pulses, and receiver frequency for future MRI images to be obtained or to a e.g. a server in order correct already obtained MRI images. The position of the region of interest of the subject may be continuously be determined and provided for the motion correction of the MR data. The motion correction of MR data may comprise prospective and/or retrospective motion correction as described above.

In another exemplary embodiment, a computer program or computer program element is provided that is configured to execute the method steps of the method according to one of the preceding embodiments, on an appropriate device or system.

The computer program element might therefore be stored on a data processing unit, which might also be part of an embodiment. This data processing unit may be configured to perform or induce performing of the steps of the method described above. Moreover, it may be configured to operate the components of the above described device and/or system. The computing unit can be configured to operate automatically and/or to execute the orders of a user. A computer program may be loaded into a working memory of a data processor. The data processor may thus be equipped to carry out the method according to one of the preceding embodiments.

Further, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that embodiments of the present disclosure are described with reference to different subject matter. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items recited in the claims. The mere fact that certain measures are re-cited in mutually different dependent claims does not indicate that a combination of these measures cannot be used to advantage. Any reference signs in the claims should not be construed as limiting the scope.

LIST OF REFERENCE SIGNS.

| 10 | system |
|----|--------|
| 11 | apparatus |
| 12 | MRI controller |
| 13 | bore |
| 14, 15, 16 | coils |
| 17, 18 | movable bed |
| 19 | head coil |
| 20, 25 | first camera |
| 21 | second camera |
| 22 | MRI system |
| 23 | subject |
| 24 | region of interest |
| 26 | mirror |
| 27 | landmark |
| 50 | apparatus |

| | |
|---|---|
| 51 | generating unit |
| 52 | obtaining unit |
| 53 | first determining unit |
| 54 | second determining unit |
| 55 | providing unit |
| S10 | generating a 3D model |
| S20 | obtaining a 2D image |
| S30 | determining at least one landmark |
| S40 | determining a 3D position of a region of interest in MRI system |
| S50 | providing a 3D position of a region of interest for motion correction MR data |

**Claims**

1. A method for motion correction of MR data, comprising:

   generating, by a calculation unit (51), a three-dimensional model, 3D model, of a region of interest (24) of a subject (23) comprising at least one landmark (27) inherent to the subject (23) (S10);
   obtaining, by a first measuring device (20, 25, 52), a two-dimensional image, 2D image, of at least a part of the subject (23) inside a MRI system (22), wherein the measuring device is arranged at least partially inside a bore of the MRI system (22) (S20);
   determining, by the calculation unit (53), at least one landmark (27) in the 2D image, wherein the at least one landmark (27) in the 2D image corresponds to the at least one landmark (27) of the 3D model (S30);
   determining, by the calculation unit (54), a 3D position of the region of interest (24) of the subject (23) in the MRI system (22) based on the determined at least one landmark (27) in the 2D image (S40);
   providing, by the calculation unit (55), the 3D position of the region of interest (24) of the subject (23) for motion correction of MR data (S50),
   **characterized in that** the method further comprises:
   obtaining at least one modelling image of the subject (23), wherein the at least one modelling image is at least one image obtained by a second measuring device arranged outside the bore of the MRI system (22), and wherein the at least one modelling image is used for generating the 3D model of the region of interest (24) of the subject (23).

2. The method according to claim 1, wherein the at least one modelling image comprises 3D data of the region of interest (24) of the subject (23).

3. The method according to claim 1 or 2, wherein the second measurement device is a depth camera.

4. The method according to claim 1, wherein the second measuring device is at least two optical cameras.

5. The method according to claim 1, wherein the second measuring device is one optical camera and the step of generating the 3D model of the region of interest (24) of the subject (23) comprises aligning the modelling image of the subject (23) with a default 3D model.

6. The method according to any one of the preceding claims, wherein the generating of the 3D model is based on a machine learning system representing a mathematical algorithm processing at least one landmark (27) of a region of interest (24) of a subject (23), wherein the machine learning system is trained to describe a relation between geometrical data of a region of interest (24) of a subject (23) and at least one landmark (27) of the region of interest (24) of the subject (23).

7. The method according to any one of the preceding claims, wherein the determining of the position of region of interest (24) of the subject (23) in the MRI system (22) comprises determining of one or more rotations and one or more translations of the 3D model in relation to a position of the first measuring device (20, 25) in order to obtain a projection of the 3D model that fits to the 2D image obtained by the first measuring device (20, 25) inside the bore.

8. The method according to anyone of the preceding claims, wherein the first measuring device is arranged in a coil.

9. The method according to anyone of the preceding claims, wherein the first measuring (20, 25) device is an optical camera.

10. The method according to anyone of the preceding claims, wherein the region of interest (24) of the subject (23) is a head of the subject (23).

11. The method according to claim 1, wherein the generating the 3D model of the subject (23) is based on an anatomical model, preferably a 3D morphable model.

12. The method according to anyone of the preceding claims, wherein the position of the region of interest (24) of the subject (23) is continuously determined and provided for the motion correction of the MR data.

**13.** An apparatus (11) for motion correction of MR data, comprising:

a generating unit, configured to generate a 3D model of a subject (23) comprising at least one landmark (27) inherent to the subject (23);

an obtaining unit, configured to obtain a 2D image of at least a part of the subject (23) inside a MRI system (22), wherein the obtaining unit is arranged inside a bore of the MRI system (22);

a first determining unit, configured to determine at least one landmark (27) in the 2D image, wherein the at least one landmark (27) in the 2D image corresponds to the at least one landmark (27) of the 3D model;

a second determining unit, configured to determine a position of the subject (23) in the MRI system (22) based on the determined at least one landmark (27) in the 2D image;

a providing unit, configured to provide the position of the subject (23) for a motion correction of MR data,

**characterized in that**

the apparatus further comprises a second measuring device arranged outside the bore of the MRI system (22), configured to obtain at least one modelling image of the subject (23); and the generating unit is configured to generate the 3D model of the subject (23) using the at least one modelling image.

**14.** A system (10) for medical imaging, comprising:

an apparatus (11) according to claim 13;
an MRI system (22); and
a first camera (20, 25), configured to obtain a 2D image inside a MRI system (22).

**15.** A computer program element, which when executed by a processor is configured to carry out the steps of the method according to any one of claims 1 to 12.

**Patentansprüche**

**1.** Verfahren zur Bewegungskorrektur von MR-Daten, umfassend:

Erzeugen, durch eine Berechnungseinheit (51), eines dreidimensionalen Modells, 3D-Modell, eines Bereichs von Interesse (24) einer Person (23), der mindestens einen der Person (23) inhärenten Markierungspunkt (27) umfasst (S10);

Gewinnen, durch eine erste Messvorrichtung (20, 25, 52), eines zweidimensionalen Bildes, 2D-Bild, von mindestens einem Teil der Person (23) innerhalb eines MRT-Systems (22), wobei die Messvorrichtung zumindest teilweise innerhalb einer Bohrung des MRT-Systems (22) angeordnet ist (S20);

Bestimmen, durch die Berechnungseinheit (53), mindestens eines Markierungspunkts (27) im 2D-Bild, wobei der mindestens eine Markierungspunkt (27) im 2D-Bild dem mindestens einen Markierungspunkt (27) des 3D-Modells entspricht (S30);

Bestimmen, durch die Berechnungseinheit (54), einer 3D-Position des Bereichs von Interesse (24) der Person (23) im MRT-System (22) anhand des mindestens einen im 2D-Bild bestimmten Markierungspunkts (27) (S40);

Bereitstellen der 3D-Position des Bereichs von Interesse (24) der Person (23) durch die Berechnungseinheit (55) zur Bewegungskorrektur der MR-Daten (S50),

**dadurch gekennzeichnet, dass** das Verfahren weiter Folgendes umfasst:

Gewinnen mindestens eines Modellierungsbildes der Person (23), wobei es sich bei dem mindestens einen Modellierungsbild um ein Bild handelt, das mit einer zweiten, außerhalb der Bohrung des MRT-Systems (22) angeordneten Messvorrichtung gewonnen wurde, und wobei das mindestens eine Modellierungsbild zur Erzeugung des 3D-Modells des Bereichs von Interesse (24) der Person (23) verwendet wird.

**2.** Verfahren nach Anspruch 1, wobei das mindestens eine Modellierungsbild 3D-Daten des Bereichs von Interesse (24) der Person (23) umfasst.

**3.** Verfahren nach Anspruch 1 oder 2, wobei es sich bei der zweiten Messvorrichtung um eine Tiefenkamera handelt.

**4.** Verfahren nach Anspruch 1, wobei die zweite Messvorrichtung aus mindestens zwei optischen Kameras besteht.

**5.** Verfahren nach Anspruch 1, wobei es sich bei der zweiten Messvorrichtung um eine optische Kamera handelt und der Schritt des Erzeugens des 3D-Modells des Bereichs von Interesse (24) der Person (23) das Ausrichten des Modellierungsbildes der Person (23) mit einem Standard-3D-Modell umfasst.

**6.** Verfahren nach einem der vorstehenden Ansprüche, wobei die Erzeugung des 3D-Modells auf einem System für maschinelles Lernen basiert, das einen mathematischen Algorithmus darstellt, der mindestens einen Markierungspunkt (27) eines Bereichs von Interesse (24) einer Person (23) verarbeitet, wobei das System für maschinelles Lernen darauf trainiert ist, eine Beziehung zwischen geometrischen Daten eines Bereichs von Interesse (24) einer Person (23) und mindestens einem Markierungspunkt (27) des Bereichs von Interesse (24) der Person (23) zu beschreiben.

7. Verfahren nach einem der vorstehenden Ansprüche, wobei die Bestimmung der Position des Bereichs von Interesse (24) der Person (23) im MRT-System (22) die Bestimmung einer oder mehrerer Rotationen und einer oder mehrerer Translationen des 3D-Modells in Bezug auf eine Position der ersten Messvorrichtung (20, 25) umfasst, um eine Projektion des 3D-Modells zu gewinnen, die zu dem von der ersten Messvorrichtung (20, 25) innerhalb der Bohrung gewonnenen 2D-Bild passt.

8. Verfahren nach einem der vorstehenden Ansprüche, wobei die erste Messvorrichtung in einer Spule angeordnet ist.

9. Verfahren nach einem der vorstehenden Ansprüche, wobei es sich bei der ersten Messvorrichtung (20, 25) um eine optische Kamera handelt.

10. Verfahren nach einem der vorstehenden Ansprüche, wobei der Bereich von Interesse (24) der Person (23) ein Kopf der Person (23) ist.

11. Verfahren nach Anspruch 1, wobei die Erzeugung des 3D-Modells der Person (23) auf einem anatomischen Modell, vorzugsweise einem 3D-morphbaren Modell, basiert.

12. Verfahren nach einem der vorstehenden Ansprüche, wobei die Position des Bereichs von Interesse (24) der Person (23) kontinuierlich bestimmt wird und zur Bewegungskorrektur der MR-Daten bereitgestellt wird.

13. Einrichtung (11) zur Bewegungskorrektur von MR-Daten, umfassend:

    eine Erzeugungseinheit, die so konfiguriert ist, dass sie ein 3D-Modell einer Person (23) erzeugt, das mindestens einen der Person (23) inhärenten Markierungspunkt (27) umfasst;
    eine Gewinnungseinheit, die so konfiguriert ist, dass sie ein 2D-Bild von mindestens einem Teil der Person (23) innerhalb eines MRT-Systems (22) gewinnt, wobei die Gewinnungseinheit innerhalb einer Bohrung des MRT-Systems (22) angeordnet ist;
    eine erste Bestimmungseinheit, die so konfiguriert ist, dass sie mindestens einen Markierungspunkt (27) im 2D-Bild bestimmt, wobei der mindestens eine Markierungspunkt (27) im 2D-Bild dem mindestens einen Markierungspunkt (27) des 3D-Modells entspricht;
    eine zweite Bestimmungseinheit, die so konfiguriert ist, dass sie eine Position der Person (23) im MRT-System (22) anhand des bestimmten mindestens einen Markierungspunkts (27) im 2D-Bild bestimmt;
    eine Bereitstellungseinheit, die so konfiguriert ist, dass sie die Position der Person (23) für eine Bewegungskorrektur von MR-Daten bereitstellt, **dadurch gekennzeichnet, dass**
    die Einrichtung weiter eine zweite Messvorrichtung umfasst, die außerhalb der Bohrung des MRT-Systems (22) angeordnet ist und so konfiguriert ist, dass sie mindestens ein Modellbild der Person (23) gewinnt; und
    die Erzeugungseinheit so konfiguriert ist, dass sie das 3D-Modell der Person (23) unter Verwendung des mindestens einen Modellierungsbildes erzeugt.

14. System (10) für die medizinische Bildgebung, umfassend:

    eine Einrichtung (11) nach Anspruch 13,
    ein MRT-System (22); und
    eine erste Kamera (20, 25), die so konfiguriert ist, dass sie ein 2D-Bild innerhalb eines MRI-Systems (22) gewinnt.

15. Computerprogrammelement, das bei Ausführung durch einen Prozessor so konfiguriert ist, dass es die Schritte des Verfahrens nach einem der Ansprüche 1 bis 12 ausführt.

**Revendications**

1. Procédé de correction de mouvement de données de MR, comprenant :

    la génération, par une unité de calcul (51), d'un modèle tridimensionnel, modèle 3D, d'une région d'intérêt (24) d'un sujet (23) comprenant au moins un point de repère (27) inhérent au sujet (23) (S10) ;
    l'obtention, par un premier dispositif de mesure (20, 25, 52), d'une image bidimensionnelle, image 2D, d'au moins une partie du sujet (23) à l'intérieur d'un système d'IRM (22), dans lequel le dispositif de mesure est agencé au moins partiellement à l'intérieur d'un alésage du système d'IRM (22) (S20) ;
    la détermination, par l'unité de calcul (53), d'au moins un point de repère (27) dans l'image 2D, dans lequel le au moins un point de repère (27) dans l'image 2D correspondent à le au moins un point de repère (27) du modèle 3D (S30) ;
    la détermination, par l'unité de calcul (54), d'une position 3D de la région d'intérêt (24) du sujet (23) dans le système d'IRM (22) sur la base du au moins un point de repère déterminé (27) dans l'image 2D (S40) ;

la fourniture, par l'unité de calcul (55), de la position 3D de la région d'intérêt (24) du sujet (23) pour une correction de mouvement de données de MR (S50),

**caractérisé en ce que** le procédé comprend en outre :

l'obtention d'au moins une image de modélisation du sujet (23), dans lequel la au moins une image de modélisation est au moins une image obtenue par un second dispositif de mesure agencé à l'extérieur de l'alésage du système d'IRM (22), et dans lequel la au moins une image de modélisation est utilisée pour générer le modèle 3D de la région d'intérêt (24) du sujet (23).

2. Procédé selon la revendication 1, dans lequel la au moins une image de modélisation comprend des données 3D de la région d'intérêt (24) du sujet (23).

3. Procédé selon la revendication 1 ou 2, dans lequel le second dispositif de mesure est une caméra de profondeur.

4. Procédé selon la revendication 1, dans lequel le second dispositif de mesure comporte au moins deux caméras optiques.

5. Procédé selon la revendication 1, dans lequel le second dispositif de mesure est une caméra optique et l'étape de génération du modèle 3D de la région d'intérêt (24) du sujet (23) comprend l'alignement de l'image de modélisation du sujet (23) sur un modèle 3D par défaut.

6. Procédé selon l'une quelconque des revendications précédentes, dans lequel la génération du modèle 3D est basée sur un système d'apprentissage automatique représentant un algorithme mathématique traitant au moins un point de repère (27) d'une région d'intérêt (24) d'un sujet (23), dans lequel le système d'apprentissage automatique est entraîné à décrire une relation entre des données géométriques d'une région d'intérêt (24) d'un sujet (23) et au moins un point de repère (27) de la région d'intérêt (24) du sujet (23).

7. Procédé selon l'une quelconque des revendications précédentes, dans lequel la détermination de la position d'une région d'intérêt (24) du sujet (23) dans le système d'IRM (22) comprend la détermination d'une ou de plusieurs rotations et d'une ou de plusieurs translations du modèle 3D par rapport à une position du premier dispositif de mesure (20, 25) afin d'obtenir une projection du modèle 3D qui correspond à l'image 2D obtenue par le premier dispositif de mesure (20, 25) à l'intérieur de l'alésage.

8. Procédé selon l'une quelconque des revendications précédentes, dans lequel le premier dispositif de mesure est agencé dans une bobine.

9. Procédé selon l'une quelconque des revendications précédentes, dans lequel le premier dispositif de mesure (20, 25) est une caméra optique.

10. Procédé selon l'une quelconque des revendications précédentes, dans lequel la région d'intérêt (24) du sujet (23) est la tête du sujet (23).

11. Procédé selon la revendication 1, dans lequel la génération du modèle 3D du sujet (23) est basée sur un modèle anatomique, de préférence un modèle morphable 3D.

12. Procédé selon l'une quelconque des revendications précédentes, dans lequel la position de la région d'intérêt (24) du sujet (23) est déterminée en continu et fournie pour la correction de mouvement des données de MR.

13. Appareil (11) pour une correction de mouvement de données de MR, comprenant :

une unité génératrice, configurée pour générer un modèle 3D d'un sujet (23) comprenant au moins un point de repère (27) inhérent au sujet (23) ;

une unité d'obtention, configurée pour obtenir une image 2D d'au moins une partie du sujet (23) à l'intérieur d'un système d'IRM (22), dans lequel l'unité d'obtention est agencée à l'intérieur d'un alésage du système d'IRM (22) ;

une première unité de détermination, configurée pour déterminer au moins un point de repère (27) dans l'image 2D, dans lequel le au moins un point de repère (27) dans l'image 2D correspond à le au moins un point de repère (27) du modèle 3D ;

une seconde unité de détermination, configurée pour déterminer une position du sujet (23) dans le système d'IRM (22) sur la base du au moins un point de repère déterminé (27) dans l'image 2D ;

une unité de fourniture, configurée pour fournir la position du sujet (23) pour une correction de mouvement de données de MR,

**caractérisé en ce que**

l'appareil comprend en outre un second dispositif de mesure agencé à l'extérieur de l'alésage du système d'IRM (22), configuré pour obtenir au moins une image de modélisation du sujet (23) ; et

l'unité de génération est configurée pour générer le modèle 3D du sujet (23) à l'aide de la au moins une image de modélisation.

**14.** Système (10) pour une imagerie médicale, comprenant :

un appareil (11) selon la revendication 13 ;
un système d'IRM (22) ; et
une première caméra (20, 25), configurée pour obtenir une image 2D à l'intérieur d'un système d'IRM (22).

**15.** Élément de programme informatique qui, lorsqu'il est exécuté par un processeur, est configuré pour réaliser les étapes du procédé selon l'une quelconque des revendications 1 à 12.

**FIG. 1**

EP 4 359 811 B1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20180325415 A1

**Non-patent literature cited in the description**

• **A. KYME et al.** Marker-free optical stereo motion tracking for in-bore MRI and PET-MRI application. *Medical Physics*, 01 June 2020, vol. 47 (8)

• **A. KYME et al.** Markerless motion tracking of awake animals in positron emission tomography. *IEEE transactions on medical imaging*, 01 November 2014, vol. 33 (11)